(19) Europäisches Patentamt / European Patent Office / Office européen des brevets

(11) EP 4 067 934 B1

(12) **EUROPEAN PATENT SPECIFICATION**

(45) Date of publication and mention
of the grant of the patent:
**17.06.2026 Bulletin 2026/25**

(21) Application number: **21166154.1**

(22) Date of filing: **31.03.2021**

(51) International Patent Classification (IPC):
*G01S 7/539* (2006.01)   *G01S 15/42* (2006.01)
*G01S 15/10* (2006.01)   *G01S 15/96* (2006.01)
*G01S 15/66* (2006.01)   *A01K 29/00* (2006.01)
*A01K 61/60* (2017.01)   *A01K 61/95* (2017.01)

(52) Cooperative Patent Classification (CPC):
**G01S 7/539; A01K 29/005; A01K 61/60;
A01K 61/95; G01S 15/108; G01S 15/42;
G01S 15/66; G01S 15/96**

(54) **AQUATIC ANIMAL SHAPE CALCULATION DEVICE AND METHOD**

VORRICHTUNG UND VERFAHREN ZUR BERECHNUNG DER FORM EINES WASSERTIERS

DISPOSITIF ET PROCÉDÉ DE CALCUL DE LA FORME D'UN ANIMAL AQUATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.10.2022 Bulletin 2022/40**

(73) Proprietor: **FURUNO ELECTRIC CO., LTD.
Nishinomiya-City, Hyogo 662-8580 (JP)**

(72) Inventor: **ITO, Masanori
Nishinomiya-city, Hyogo 662-8580 (JP)**

(74) Representative: **Cleveland Scott York
5 Norwich Street
London EC4A 1DR (GB)**

(56) References cited:
**EP-A1- 3 754 374    WO-A1-2017/163904**

• ITO M ET AL: "Classification of fish schools
based on acoustic features associated with tilt
angle", UNDERWATER TECHNOLOGY
SYMPOSIUM (UT), 2013 IEEE INTERNATIONAL,
IEEE, 5 March 2013 (2013-03-05), pages 1 - 4,
XP032410874, ISBN: 978-1-4673-5948-1, DOI:
10.1109/UT.2013.6519865
• ITO MASANORI ET AL: "Target strength spectra
of tracked individual fish in schools", FISHERIES
SCIENCE, JAPANESE SOCIETY OF SCIENTIFIC
FISHERIES, JP, vol. 81, no. 4, 29 May 2015
(2015-05-29), pages 621 - 633, XP035514555,
ISSN: 0919-9268, [retrieved on 20150529], DOI:
10.1007/S12562-015-0890-7

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present disclosure relates to signal processing in an aquaculture environment, and more particularly to aquatic animal shape calculation using ultrasonic device.

In an aquaculture environment, such as a fish farm, aquatic animals like fish are raised in fish cages, and in order to check that the fish are raised properly, there is a need to regularly measure the size and the weight of the fish. The size and the weight of the fish may be measured by measuring an internal structure/shape of the fish. Also, it is preferable to remotely measure the internal structure/shape of the fish without actually physically touching the fish, in order not to stress or hurt the fish.

EP3754374A1 (Furuno Electric Co.), describes apparatus for measuring fish weight from calculations of fish height, length and width. "Classification of fish schools based on acoustic features associated with tilt angle" (Underwater Technology Symposium, 5 March 2013), describes a technique for classifying fish echoes, including using a broadband split-beam echo-sounder to track echoes and calculate an apparent tilt-angle of a fish. WO2017/163904A1 (Furuno Electric Co.), describes an underwater detection-apparatus comprising a split-beam echo sounder, and a method for fish species discrimination based on an incidence angle and a target strength of a fish. "Target strength spectra of tracked individual fish in schools" (Fisheries Science, Japanese society of fisheries, vol. 81, no. 4, 29 May 2015), describes a method for remote identification of fish species using an echo sounder.

As a method to remotely measure a structure/shape of the fish, an underwater camera is placed in the fish cage to acquire pictures of the fish. The shape of fish and fish size may then be calculated from the pictures. Alternatively, use of X-ray is another conventional method to measure the shape of a living creature like fish. An advantage of X-ray compared to camera is that both the internal and external structure/shape of the fish can be measured, whereas the camera can only measure the external shape of the fish. The ability to measure the internal structure/shape of the fish would likely lead to an improved fish size/weight measurement. However, it would be difficult to measure the internal structure/shape of fish with X-ray due to continuous motion of the fish, while the fish are swimming in the fish cage.

Hence, there is a need to measure the internal structure/shape of aquatic animals swimming in water using ultrasound, and thereby improve accuracy of the measurement of the fish size and weight.

In a first aspect of the present invention, there is provided a device for calculating an internal structure of an aquatic animal , the device comprising a transducer, an ultrasound incidence angle calculation module, a parts distance calculation module, and an internal structure information module. The transducer is configured to transmit first and second ultrasonic waves toward an aquatic animal and generate first and second echo signals from a reflection of the first and second ultrasonic waves on the aquatic animal, respectively. The ultrasound incidence angle calculation module is configured to calculate first and second incidence angles of the first and second ultrasonic waves on the aquatic animal based on the first and second echo signals, respectively. The parts distance calculation module is configured to calculate a first internal distance between first and second body parts of the aquatic animal based on the first echo signal, and calculate a second internal distance between first and second body parts of the aquatic animal based on the second echo signal. The internal structure information module is configured to store the first incidence angle and the first internal distance as a first pair, store the second incidence angle and the second internal distance as a second pair, and store the internal structure of the aquatic animal, wherein the internal structure is based on the first and second pairs.

Additionally, the second ultrasonic wave is transmitted after the first ultrasonic wave, and the ultrasound incidence angle calculation module calculates the second incidence angle based on a first distance between the aquatic animal and the transducer in the first echo signal and a second distance between the aquatic animal and the transducer in the second echo signal.

Additionally, the ultrasound incidence angle calculation module calculates the second incidence angle based on an assumption that the first and second distances change with time based on a hyperbolic curve.

Additionally, the ultrasound incidence angle calculation module calculates the first incidence angle based on another distance between the aquatic animal and the transducer in another echo signal and the first distance between the aquatic animal and the transducer in the first echo signal, the other echo signal being generated from a reflection of another ultrasonic wave, transmitted before the first ultrasonic wave, on the aquatic animal; and the ultrasonic incidence angle calculation module calculates the first incidence angle based on an assumption that the other distance and the first distance change with time based on a hyperbolic curve.

Additionally, or optionally, the device further comprises an individual weight calculation module configured to calculate a weight of the aquatic animal, based on the internal structure of the aquatic animal, by multiplying the first internal distance raised to the power of a first predetermined value, with the second internal distance raised to the power of a second predetermined value.

Additionally, or optionally, the transducer is configured to transmit the first and second ultrasonic waves toward a plurality of aquatic animals including the aquatic animal. The ultrasound incidence angle calculation module is configured to calculate the first and second incidence angles for the plurality of aquatic animals. The parts distance calculation module is configured to calculate the first and second internal distances for the plurality of aquatic animals. The internal structure information module is configured to store the internal structure of the plurality of aquatic animals.

Additionally, or optionally, the device further comprises a histogram calculation module configured to calculate a histogram representing a distribution of incidence angle and internal distance of the plurality of aquatic animals from the internal structure of the plurality of aquatic animals. The internal structure information module is further configured to store the histogram as a global internal structure of the plurality of aquatic animals.

Additionally, or optionally, the device further comprises a mean weight calculation module configured to calculate a mean weight of the plurality of aquatic animals from the histogram by extracting from the histogram a plurality of internal distances at a corresponding plurality of incidence angles, and by calculating the mean weight based on the extracted plurality of internal distances.

Additionally, or optionally, the mean weight calculation module calculates the mean weight based on a multiplication of the plurality of internal distances, each raised to the power of a predetermined value.

Additionally, or optionally, the device further comprises a motor coupled to the transducer, and configured to move the transducer to change a direction in which the transducer transmits the first and second ultrasonic waves. The transducer transmits the first ultrasonic wave in a first direction, and the second ultrasonic wave in a second direction different from the first direction.

Additionally, or optionally, the transducer comprises first and second transducer elements spaced apart by a predetermined distance, and wherein the first transducer element transmits the first ultrasonic wave and the second transducer element transmits the second ultrasonic wave.

Additionally, or optionally, the first and second transducer elements transmit the first and second ultrasonic waves in directions that are different with respect to each other, respectively.

In another aspect of the present invention, there is provided a method for calculating an internal structure of an aquatic animal the method including transmitting first and second ultrasonic waves toward an aquatic animal, generating first and second echo signals from a reflection of the first and second ultrasonic waves on the aquatic animal, respectively, calculating first and second incidence angles of the first and second ultrasonic waves on the aquatic animal based on the first and second echo signals, respectively, calculating a first internal distance between first and second body parts of the aquatic animal based on the first echo signal, calculating a second internal distance between first and second body parts of the aquatic animal based on the second echo signal, storing the first incidence angle and the first internal distance as a first pair, storing the second incidence angle and the second internal distance as a second pair, and storing an internal structure of the aquatic animal based on the first and second pairs; wherein the second ultrasonic wave is transmitted after the first ultrasonic wave; and wherein the method further comprises calculating the second incidence angle based on a first distance between the aquatic animal and a transducer in the first echo signal and a second distance between the aquatic animal and the transducer in the second echo signal; and calculating the second incidence angle based on an assumption that the first and second distances change with time based on a hyperbolic curve; and wherein the first incidence angle is calculated based on another distance between the aquatic animal and the transducer in another echo signal and the first distance between the aquatic animal and the transducer in the first echo signal, the other echo signal being generated from a reflection of another ultrasonic wave, transmitted before the first ultrasonic wave, on the aquatic animal; and the first incidence angle is calculated based on an assumption that the other distance and the first distance change with time based on a hyperbolic curve.

In yet another aspect of the present invention, there is provided a non-transitory computer readable medium having stored thereon computer-executable instructions which, when executed by a device as described above, cause the device to execute a method as described above.

The problem of not being able to measure the internal structure/shape of a moving aquatic animal, such as fish, with ultrasound is solved by calculating incidence angles of ultrasonic waves on the aquatic animal and calculating internal parts distances corresponding to the calculated incidence angles, thereby improving an accuracy of aquatic animal size/weight measurement with ultrasound.

These as well as other aspects, advantages, and alternatives will become apparent to those of ordinary skill in the art by reading the following detailed description with reference where appropriate to the accompanying drawings. Further, it should be understood that the description provided in this summary section and elsewhere in this document is intended to illustrate the claimed subject matter by way of example and not by way of limitation.

The illustrated embodiments of the subject matter will be best understood by reference to the drawings, wherein like parts are designated by like numerals throughout. The following description is intended only by way of example, and simply illustrates certain selected embodiments of devices, systems, and processes that are consistent with the subject matter as claimed herein:

**FIG. 1** is a block diagram showing an overall configuration of an aquatic animal shape calculation device, in accordance with an embodiment of the present disclosure;

**FIG. 2** is a perspective view showing the aquatic animal shape calculation device in a cage, in accordance with an embodiment of the present disclosure;

**FIG. 3** illustrates an example of an envelope of first, second, and third echo signals generated based on first, second, and third ultrasonic waves, respectively;

**FIG. 4A** illustrates a fish tracked at two positions using two consecutive transmissions by the transducer, in accordance with an embodiment of the present disclosure;

**FIG. 4B** illustrates a fish tracked at two positions using two consecutive transmissions by the transducer, in accordance with another embodiment of the present disclosure;

**FIG. 4C** illustrates a fish tracked at two positions using two consecutive transmissions by the transducer, in accordance with yet another embodiment of the present disclosure;

**FIG. 5** illustrates an example of an envelope of an echo signal generated based on an ultrasonic wave;

**FIG. 6** illustrates an internal structure of the fish obtained based on a correspondence between incidence angle and internal distance for an individually tracked fish;

**FIG. 7** is a block diagram showing an overall configuration of an aquatic animal shape calculation device, in accordance with another embodiment of the present disclosure;

**FIG. 8** illustrates an ultrasound incidence angle vs back distance relative to swimbladder histogram, in accordance with an embodiment of the present disclosure;

**FIG. 9A** illustrates a relative position of the swimbladder and the back of fish swimming in the fish cage obtained from the histogram;

**FIG. 9B** illustrates a global internal structure obtained based on a correspondence between incidence angle and the global internal distance for a plurality of fish;

**FIG. 10A** illustrates a top view showing the calculation of the incidence angle when the transducer includes a single receiving channel, in accordance with an embodiment of the present disclosure;

**FIG. 10B** illustrates a side view showing the calculation of the incidence angle when the transducer includes a single receiving channel, in accordance with an embodiment of the present disclosure;

**FIG. 11** illustrates various echo signals generated at different time instances, when the fish swims below the single receiving channel transducer;

**FIG. 12** illustrates the transducer coupled to a motor such that the transducer transmits the first and second ultrasonic waves towards the aquatic animals in different directions at different time instances;

**FIG. 13** illustrates tracking of a fish using a transducer having first and second transducer elements; and

**FIG. 14** is a flow chart illustrating an aquatic animal shape calculation method, in accordance with an embodiment of the present disclosure.

Example apparatus are described herein.. In the following detailed description, reference is made to the accompanying drawings, which form a part thereof.

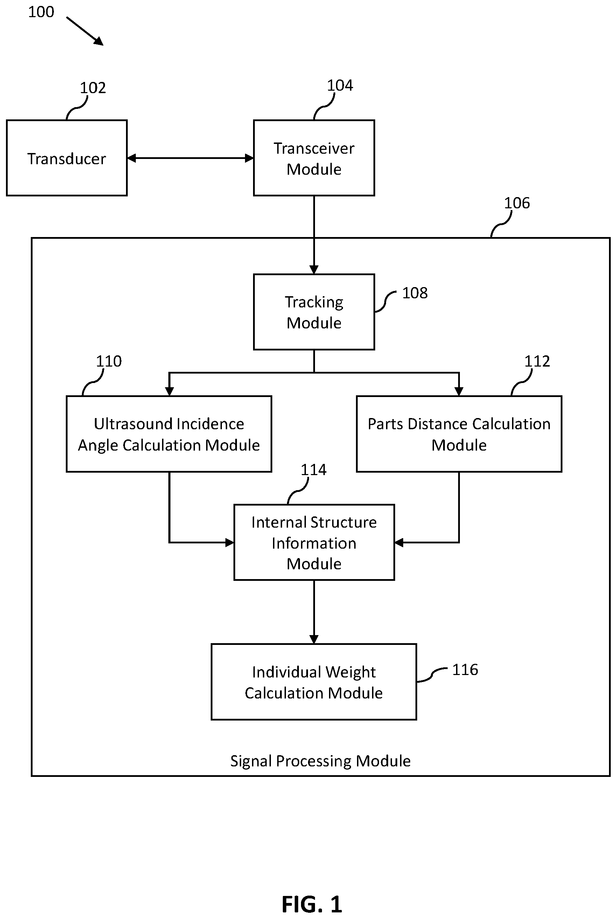

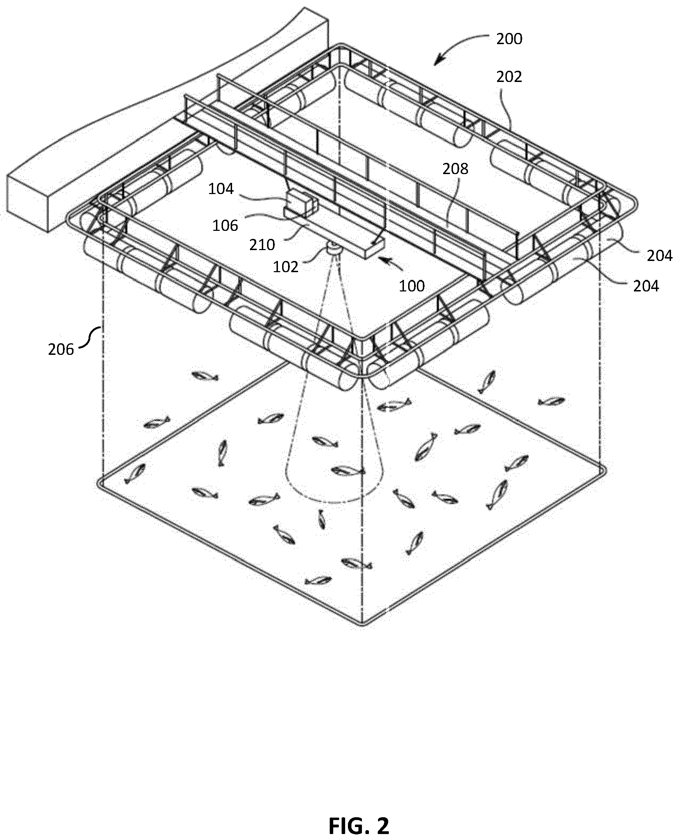

**FIG. 1** is a block diagram showing an overall configuration of an aquatic animal shape calculation device **100,** in accordance with an embodiment of the present invention. **FIG. 2** is a perspective view showing the aquatic animal shape calculation device **100** in a cage **200,** in accordance with an embodiment of the present disclosure.

The cage **200** is configured as a net-shaped cage, and includes a frame **202,** a float **204,** a net **206,** and a pier **208.** The frame **202** is formed so as to have a loop shape in a plan view. A plurality of floats **204** are attached to the frame **202,** whereby the frame **202** may be floated on a surface of the water. The frame **202** is connected to a weight at the bottom of the water by a mooring rope (not shown).

In the present embodiment, the frame **202** is formed to have the loop shape and the frame **202** is connected to the weight by the mooring rope. In various other embodiments, the frame **202** may be formed to have any suitable shape and the frame **202** may be connected to the weight by any known suitable technique.

An upper end of the net **206** is fixed to the frame **202.** The net **206** is suspended so as to partition the water to form a closed space, and fish of known species are bred inside this space. The pier **208** is fixed on the frame **202** for performing various operations related to aquaculture.

A float **210** is floated in a substantially central portion inside the frame **202,** and the float **210** is connected to the pier **208** by a rope. The aquatic animal shape calculation device **100** of the present embodiment is arranged on the float **210.** As shown in **FIG. 1,** the aquatic animal shape calculation device **100** includes a transducer **102,** a transceiver module **104,** and a signal processing module **106.**

The transducer **102** mutually converts an electric signal and ultrasonic vibration, and detects an aquatic animal underwater by using a transmitted wave which is an ultrasonic wave. In an embodiment, the transducer **102** is attached to a lower part of the float **210.** The transducer **102** is arranged to transmit ultrasonic waves downward from an upper side of the water surface toward the aquatic animal in the cage **200.** At a time of transmission, the transducer **102** converts a high power transmission signal provided by the transceiver module **104** into an ultrasonic wave and transmits the ultrasonic wave underwater. Examples of the aquatic animal include, but are not limited to, a fish, a shrimp, or a dolphin. For the sake of ongoing discussion, it is assumed that the aquatic animal is a fish and the aquatic animal may be hereinafter interchangeably referred to as a fish.

The transducer **102** receives reflected waves due to reflection of the ultrasonic waves on the aquatic animal. At a time of reception, the transducer **102** receives reflection wave reflected from underwater targets, such as the aquatic animals, converts the reflection wave into an electric signal, *i.e.,* echo signal, and outputs the corresponding echo signal back to the transceiver module **104.** One such transmission and reception cycle of the ultrasonic wave and the reflected wave is known as a ping. On completion of one transmission and reception cycle, the transmission and reception cycle of the ultrasonic wave and the reflected wave is repeated again. In one embodiment, the transducer **102** transmits first and second ultrasonic waves toward the aquatic animal such that the second ultrasonic wave is transmitted after the first ultrasonic wave, and generates first and second echo signals from a reflection of the first and second ultrasonic waves on the aquatic animal, respectively.

The transceiver module **104** is connected to the transducer **102** via an electric cable. The transceiver module **104** outputs an electric signal to the transducer **102** via the electric cable based on which the transducer **102** transmits the ultrasonic waves, such as the first and second ultrasonic waves. Further, the transceiver module **104** acquires via the electric cable the echo signals, such as the first and second echo signals, generated by the transducer **102** based on the reflected waves. Further, the transceiver module **104** may be configured to amplify and filter the corresponding echo signal, convert the corresponding echo signal (analog signal) into a reception signal which is a digital signal and store in a memory (not shown) of the signal processing module **106.**

As shown in **FIG. 1,** the signal processing module **106** includes a tracking module **108,** an ultrasound incidence angle calculation module **110,** a parts distance calculation module **112,** an internal structure information module **114,** and an individual weight calculation module **116.** The signal processing module **106** may be configured as a known computer, and may be communicatively coupled to the transceiver module **104** through a communication cable to acquire received signals from the transducer **102.** The signal processing module **106** is configured to receive, store and process the echo signals to calculate an internal structure of the aquatic animal and measure a length, breadth, height and/or weight of the aquatic animal in water. Specifically, the signal processing module **106** may include a CPU, a ROM, a RAM, and the like. A program for realizing an aquatic animal shape calculation method of the present invention may be stored in the ROM or the like.

It may be noted that the calculation of internal structure of fish is not limited to the fish raised in the fish cage **200,** for example, the aquatic animal shape calculation device **100** may be attached to a fishing boat and used as a device for estimating the internal structure for a school of fish swimming in the sea.

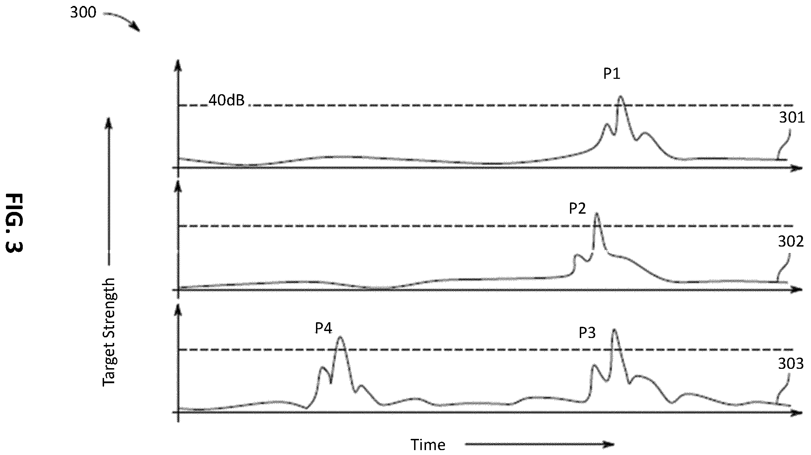

FIG. 3 illustrates an example of an envelope **300** of first, second, and third echo signals **301, 302,** and **303** generated based on first, second, and third ultrasonic waves T1, T2, and T3, respectively. The first, second, and third ultrasonic waves T1, T2, and T3 are transmitted subsequently one after another, and corresponding echo signals **301, 302,** and **303** are stored in a memory (not shown). The first, second and third ultrasonic waves may be hereinafter also referred to as first, second and third pings respectively.

In the envelope **300,** the horizontal axis is time and the vertical axis is Target Strength (TS) which indicates a strength of the received echo signal. The target strength is a parameter indicating a degree to which a part of the ultrasonic wave that is scattered by hitting the fish returns in the incident direction, and can be considered to be substantially the same as the echo intensity.

Referring to **FIGs. 1** and **3,** the tracking module **108** tracks with respect to time an aquatic animal, such as a fish, among the underwater targets from the first, second and third echo signals **301, 302** and **303** resulting from corresponding ultrasonic waves T1, T2, and T3. The tracking module **108** is configured to perform a swimbladder peak extraction in the echo signals **301, 302** and **303.** The tracking module **108** determines whether or not the target strength (TS) value is equal to or higher than a predetermined detection threshold value. A peak of an echo signal that is above the predetermined detection threshold value is considered as an echo coming from the swimbladder of a fish, and is hereinafter also referred to as a swimbladder peak. In general, it is known that the ultrasonic waves transmitted from the transducer **102** are most strongly reflected on the swimbladder among each part of the fish. Therefore, as shown in **FIG. 3,** when the target strength (TS) shows the maximum peak, this peak can be considered to be derived from the reflected wave reflected by the swimbladder. A peak of an echo signal that is below the predetermined detection threshold value corresponds to an echo from other body parts of a fish such as back of fish or belly of fish. Herein, the predetermined detection threshold value is -40dB, such that the first echo signal **301** includes a swimbladder peak P1, the second echo signal **302** includes a swimbladder peak P2, and the third echo signal **303** includes two swimbladder peaks P3 and P4.

For each swimbladder peak in a given echo signal, the tracking module **108** determines if there is a corresponding swimbladder peak for a previous transmission wave that occurred at about the same timing. If there is such a peak, the two swimbladder peak echoes are considered as actually coming from the same fish. In an example, peaks P1, P2, and P3 occur almost at the same timing, and therefore, can be considered coming from the same fish.

It would be apparent to one of ordinary skill in the art, that the tracking module **108** performs fish tracking by comparing peak positions on echo signals from two consecutive transmissions. However, there is no limitation on the number of consecutive transmissions, and more than two transmissions can be used for fish tracking. The tracking module **108** may employ sophisticated tracking algorithms for fish tracking, involving, for example, prediction by the use of Kalman filter.

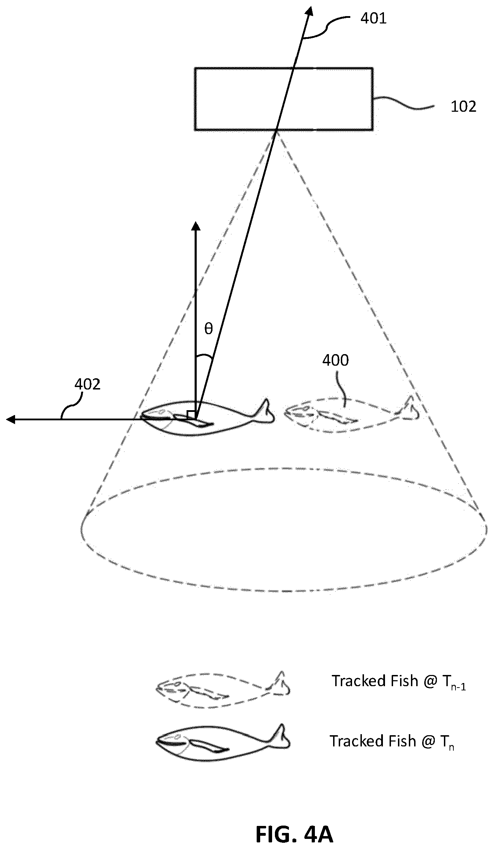

**FIG. 4A** illustrates a fish **400** tracked at two positions using two consecutive transmissions **T<sub>n-1</sub>** and **T<sub>n</sub>** by the transducer **102**, in accordance with an embodiment of the present disclosure. For each position of the tracked fish **400**, the ultrasound incidence angle calculation module **110** calculates an incidence angle θ at which the transmitted ultrasonic wave penetrates into the tracked fish **400**.

In one embodiment of the present disclosure, the transducer **102** includes one transmitter and a receiver having a plurality of elements divided into four receiving channels. Each receiving channel has a different directivity, and receives the reflected wave reflected from the target in the water. Then, the transducer **102** converts the data related to the received reflected wave into an electric signal, *i.e.*, an echo signal. The ultrasound incidence angle calculation module **110** may calculate a direction of arrival (DOA) of the echoes from the fish **400** using the conventional interferometry principle (also known as split beam method), *i.e.*, by calculating a phase difference between the echo signals received by the corresponding receiving channels of the transducer **102**. According to the conventional split beam method, an angle between the DOA of the reflected wave and a direction of the central axis of the transmitted wave is calculated based on the phase difference.

The ultrasound incidence angle calculation module **110** calculates a 3D position of the tracked fish **400** at each transmission. Particularly, for each position, the ultrasound incidence angle calculation module **110** calculates a distance between the fish 400 and the transducer **102** based on time difference between transmission and reception of the echoes of the fish **400**. Further, the ultrasound incidence angle calculation module **110** calculates the 3D position of the fish **400** relative to the transducer **102** based on corresponding DOA of the fish echo and the distance between the fish **400** and the transducer **102**. The position of the fish **400** relative to the transducer **102** constitutes a fish-transducer vector **401**, which may vary from one transmission to another. In the present embodiment, the fish-transducer vector **401** indicates the position of the fish **400** relative to the transducer **102** during the current transmission **T<sub>n</sub>**.

The ultrasound incidence angle calculation module **110** further calculates a swimming direction vector **402** of the fish **400** based on change in position of the fish **400** from the previous transmission **T<sub>n-1</sub>** to the current transmission **T<sub>n</sub>**. The ultrasound incidence angle calculation module **110** calculates the incidence angle θ by calculating an angle made between the swimming direction vector **402** (*i.e.*, a first line that connects two different positions of the tracked fish **400** in time) and the fish-transducer vector **401** (*i.e.*, a second line that connects one of the two different positions and a position of the transducer **102**).

The ultrasound incidence angle calculation module **110** calculates, for each position of the tracked fish **400**, the incidence angle θ of the tracked fish **400** based on the following equation:

$$\text{Incidence angle } \theta = 90° - \text{angle (swimming direction vector } \textbf{402}, \text{ fish-transducer vector } \textbf{401})... (1) \qquad\qquad (1)$$

The incidence angle θ is a complementary angle of an angle between the swimming direction vector **402** and the fish-transducer vector **401**. In an example, the incidence angle θ is zero for a fish that is vertically below the transducer **102**, and is swimming horizontally. It will be apparent to person skilled in the art, that there is no limitation to the definition of the incidence angle θ mentioned above, and the incidence angle θ may be defined in any suitable manner.

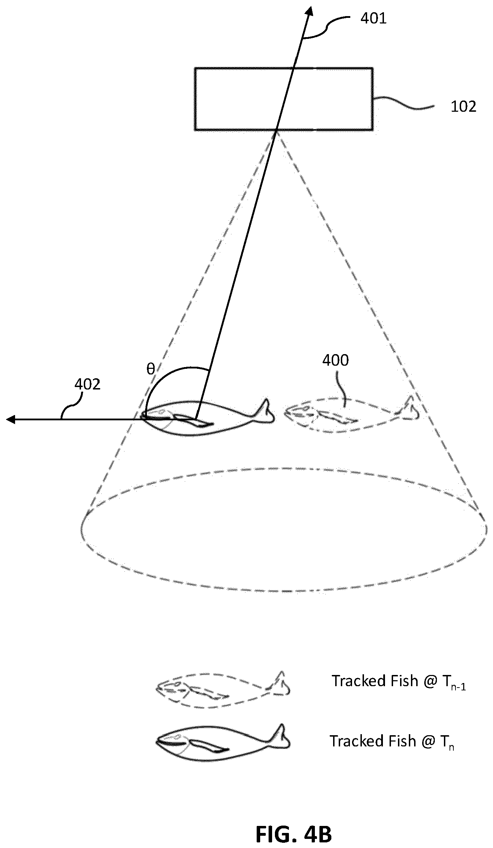

In another embodiment of the present disclosure, the incidence angle θ may be defined as an angle between the swimming direction vector **402** and the fish-transducer vector **401** as shown in **FIG. 4B**. In this embodiment, the ultrasound incidence angle calculation module **110** calculates, for each position of the tracked fish **400**, an incidence angle θ of the tracked fish **400** based on the following equation:

$$\text{Incidence angle } \boldsymbol{\theta} = \text{angle (swimming direction vector } \textbf{402}, \text{ fish-transducer vector } \textbf{401})$$

$$\ldots\ldots (2)$$

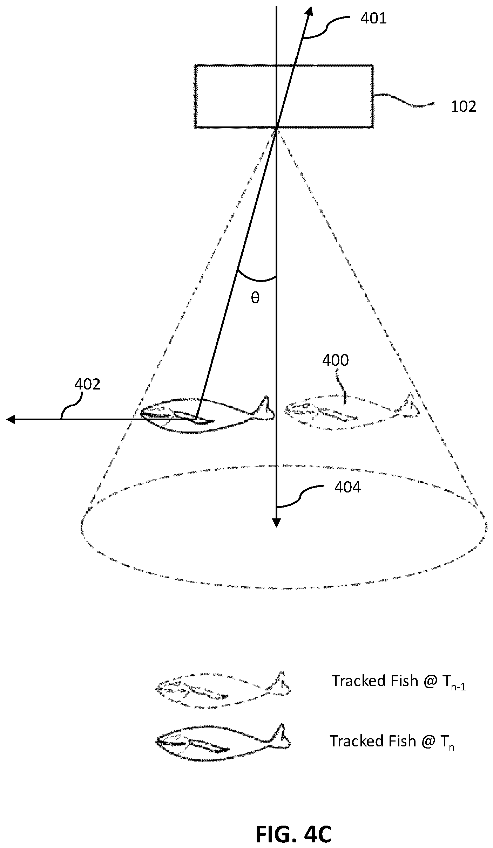

In yet another embodiment of the present disclosure, the incidence angle θ may be defined as an angle between the fish-transducer vector **401** and a vertical direction vector **404** from the transducer **102** toward bottom of the water as shown in **FIG. 4C.** In this embodiment, the ultrasound incidence angle calculation module **110** calculates, for each position of the tracked fish **400**, an incidence angle θ of the tracked fish **400** based on the following equation:

$$\text{Incidence angle } \boldsymbol{\theta} = \text{angle (fish-transducer vector } \textbf{401}, \text{ vertical direction vector } \textbf{404})$$

$$\ldots\ldots\ldots\ldots (3)$$

Thus, the ultrasound incidence angle calculation module **110** calculates the incidence angle θ for each transmission (ping) based on corresponding echo signal. For example, the ultrasound incidence angle calculation module **110** calculates first and second incidence angles $\theta_1$ and $\theta_2$ of the first and second transmissions $T_n$ and $T_{n-1}$ on the fish **400** based on corresponding first and second echo signals. The ultrasound incidence angle calculation module **110** calculates the first incidence angle $\theta_1$ based on calculation of a first position of the fish **400** within a beam of the transducer **102,** based on the first echo signal corresponding to first transmission $T_n$, and the ultrasound incidence angle calculation module **110** calculates the second incidence angle $\theta_2$ based on calculation of a second position of the fish **400** within a beam of the transducer **102,** based on the second echo signal corresponding to second transmission $T_{n-1}$. The ultrasound incidence angle calculation module **110** calculates the first and second positions using interferometry (conventional split beam method) as described above.

Although in the present embodiment, it is assumed that the transducer **102** transmits the ultrasonic waves vertically downward from above the fish **400,** in various other embodiments, the transducer **102** may also be arranged to transmit the ultrasonic waves vertically upward from below the fish **400.**

In the embodiment mentioned above, the transducer **102** includes a plurality of receiving channels and the incidence angle $\theta$ is calculated based on a calculation of a position of the fish within a beam of the transducer **102** using the conventional split beam method. In an alternate embodiment of the present disclosure, the transducer **102** includes a single receiving channel and the incidence angle $\theta$ may be calculated using a suitable method. The calculation of the incidence angle $\theta$ when the transducer **102** includes the single receiving channel has been explained later.

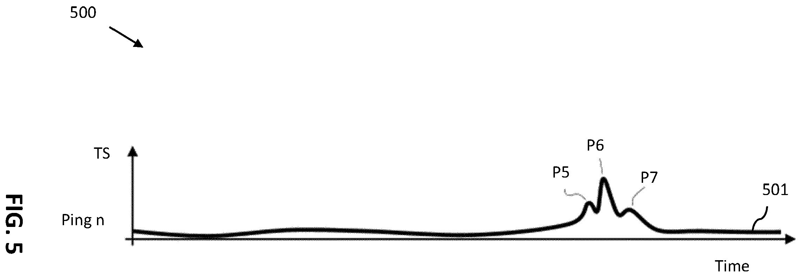

**FIG. 5** illustrates an example of an envelope **500** of an echo signal **501** generated based on a transmission $T_n$ (ping n) of ultrasonic wave. In the echo signal **501,** several peaks appear depending on the fish species and the maturity of the fish **400.** In one example, three peaks P5, P6, P7 are illustrated, where P5 corresponds to an echo reflected from the back of the fish **400,** P6 corresponds to an echo reflected from a swimbladder of the fish **400,** and P7 corresponds to an echo reflected from a belly of the fish **400.** It will be understood by a person skilled in the art that depending on the fish species and maturity of the fish **400,** it is also possible to have more than three peaks. For example, a peak corresponding to an echo reflected from the backbone of the fish **400,** another peak corresponding to an echo reflected from the head bone of the fish **400** may also be present in the echo signal **501.**

In order to detect the echo reflected from the back of the fish **400,** the following processing may be performed. When the transducer **102** transmits the ultrasonic waves downward from above the fish **400,** the echo with a maximum echo intensity, *i.e.,* a target strength (TS), is considered as an echo reflected from the swimbladder of the fish **400** and a peak in the echo signal appearing immediately before that of the swimbladder peak is considered as an echo from the back of the fish **400.** In order to make sure with a high reliability that the peak before the swimbladder peak is actually the peak corresponding to the back of the fish **400,** a processing circuit (not shown) of the parts distance calculation module **112** may check the following condition. The processing checks within a given time interval of a given duration, extending before the peak corresponding to the back of the fish and starting from the peak corresponding to the back of the fish, whether another peak of at least a given echo intensity exists. If no such other peak exists within that given time interval, the peak appearing immediately before the swimbladder peak is considered as corresponding to the back of the fish with a high reliability.

Referring to **FIGs. 1** and **5,** for an echo signal such as the echo signal **501** generated based on the transmission $T_n$ (ping n) of the ultrasonic wave, the parts distance calculation module **112** calculates a time difference between two peaks in the echo signal **501** and then multiply the time difference with the speed of ultrasonic waves in the water (typically, $1500 m.s^{-1}$) to generate an internal distance between the corresponding body parts of the fish. In an example, the parts distance calculation module **112** multiplies a time difference between peaks P5 and P6 with the speed to determine an internal distance between the back and swimbladder of the fish **400,** multiplies a time difference between peaks P6 and P7 with the speed to determine an internal distance between the belly and swimbladder of the fish **400,** and so on.

In the context of the present disclosure, the parts distance calculation module **112** calculates an internal distance between first and second body parts of the aquatic animal for each echo signal. It will be understood by a person skilled in the art the parts distance calculation module **112** may calculate the internal distance between any two internal parts of the fish **400,** such as the back and belly, the back and swimbladder, and the belly and swimbladder for various positions of the tracked fish **400.** For the sake of ongoing discussion, it is assumed that the first body part is assumed to be swimbladder, and the second body part is assumed to be back of the fish, and the internal distance is the distance between the back and the swimbladder of the fish, and may be hereinafter also referred to as swimbladder-back distance.

Thus, for the first transmission $T_n$ of the ultrasonic wave, the parts distance calculation module **112** calculates a first internal distance $d_1$ between the first and second body parts, and for the second transmission $T_{n-1}$, the parts distance calculation module **112** calculates a second internal distance $d_2$ between first and second body parts of the aquatic animal.

For each position of the tracked fish **400,** the internal structure information module **114** stores a correspondence between the calculated internal distance and corresponding incidence angle, such that, the correspondence between the calculated internal distance and the corresponding incidence angle for a plurality of incidence angles is representative of an internal structure of the aquatic animal, *i.e.,* the tracked fish **400.** The internal structure information module **114** stores the first incidence angle $\theta_1$ and the first internal distance $d_1$ as a first pair, the second incidence angle $\theta_2$ and the second internal distance $d_2$ as a second pair, and so on, for example, in form of a table illustrated below:

**Table I**

| Tracked fish | |
| --- | --- |
| Incidence angle | Internal distance |
| $\theta_1$ | $d_1$ |
| $\theta_2$ | $d_2$ |
| $\theta_3$ | $d_3$ |

Table I illustrates three incidence angles and three internal distances calculated at three different pings. In various other embodiments, 'n' incidence angles and 'n' internal distances may be calculated at 'n' different pings, where 'n' is any integer greater than two, as illustrated below:

**Table II**

| Tracked fish | |
| --- | --- |
| Incidence angle | Internal distance |
| $\theta_1$ | $d_1$ |
| ... | ... |
| $\theta_n$ | $d_n$ |

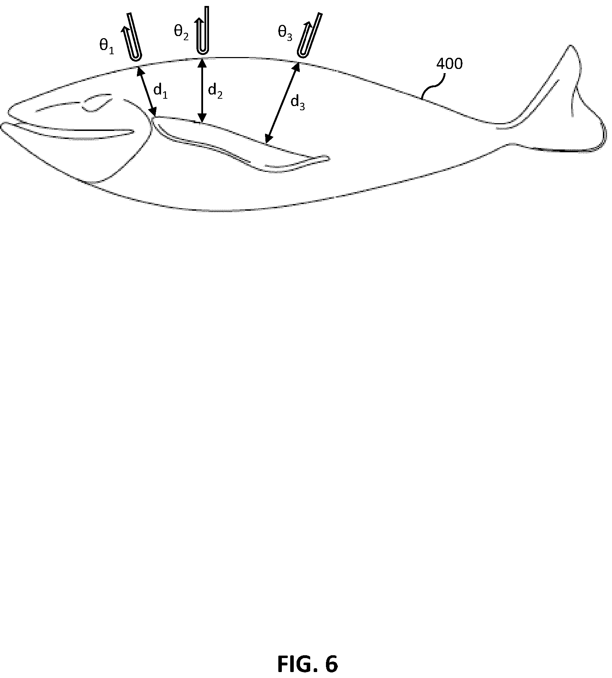

**FIG. 6** illustrates the tracked fish 400 and internal distances $d_1$, $d_2$, and $d_3$ calculated at different incidence angles $\theta_1$, $\theta_2$, and $\theta_3$ across three pings (three transmissions). The internal structure information module **114** stores the internal structure of the aquatic animal, *i.e.,* the fish **400,** in the form of Table I or II.

The individual weight calculation module **116** calculates a weight of the fish **400** based on the internal structure of the aquatic animal. In the context of the present disclosure, when first and second internal distances $d_1$ and $d_2$ are calculated for the tracked fish **400,** the individual weight calculation module **116** calculates the weight of the fish **400,** by multiplying the first internal distance $d_1$ raised to the power of a first predetermined value $\beta_1$, with the second internal distance $d_2$ raised to the power of a second predetermined value $\beta_2$ and a third predetermined value $\beta_0$. The weight of the fish **400** is represented as follows:

$$Weight = \beta_0 * d_1^{\beta_1} * d_2^{\beta_2}$$

where the first, second and third predetermined values $\beta_0$, $\beta_1$, and $\beta_2$ correspond to a particular species of fish.

For example, for the individual fish **400,** tracked 'N' times, for which the corresponding 'N' incidence angles $\theta_1$, ..., $\theta_n$ and 'N' internal distances $d_1$, ..., $d_n$ are calculated, a weight $W_i$ of the individual fish i may be calculated as follows:

$$Wi = \beta_0 \prod_{1}^{N} d_n^{\beta_n}$$

where $\beta_n$ ($0 \leq n \leq N$) are predetermined values based on experiment data for a given fish species. Thus, the individual weight calculation module **116** calculates the fish weight for an individually tracked fish.

In the current embodiment, the internal structure and the weight of an individual fish, such as the fish **400,** is determined. In various other embodiments, the internal structure and the weight of a plurality of fish may be determined.

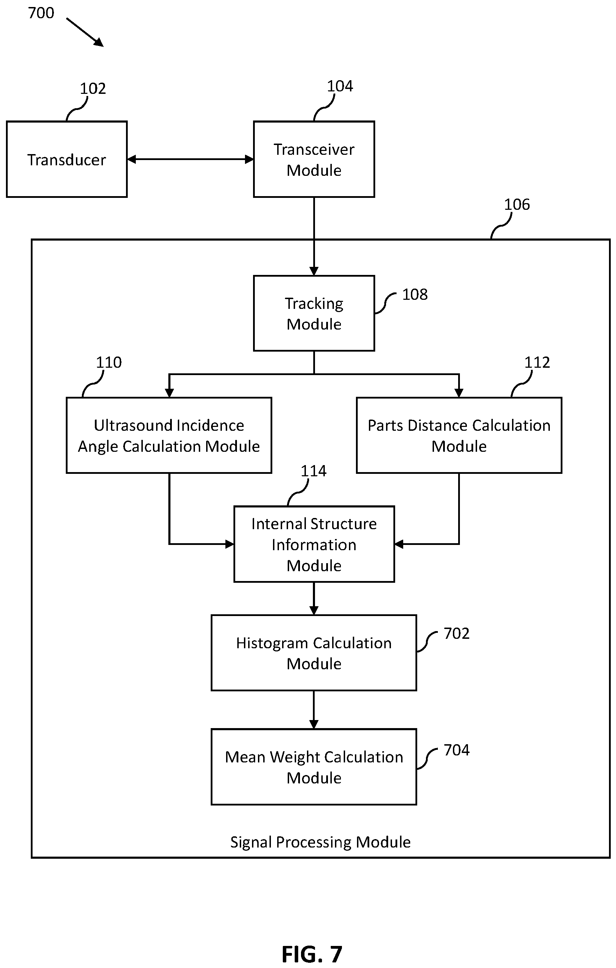

**FIG. 7** is a block diagram showing an overall configuration of an aquatic animal shape calculation device **700,** in accordance with another embodiment of the present disclosure.

The aquatic animal shape calculation device **700** of **FIG. 7** differs from the aquatic animal shape calculation device **100** of **FIG. 1** in the configuration of a histogram calculation module **702** and a mean weight calculation module **704.** Also, the aquatic animal shape calculation device **700** does not include the individual weight calculation module **116** of **FIG. 1.** It will be understood by a person skilled in the art that the aquatic animal shape calculation device **700** may be arranged on the float **210** of the cage **200** in a manner similar to the arrangement of the aquatic animal shape calculation device **100** of **FIG. 1.**

In the present embodiment, the aquatic animal shape calculation device 700 does not include the individual weight calculation module **116** of **FIG. 1.** In various other embodiments, the aquatic animal shape calculation device **700** may include the individual weight calculation module **116** of **FIG. 1.**

Referring now to **FIG. 7,** the transducer **102** transmits a plurality of ultrasonic waves toward a plurality of aquatic animals, similar to the fish **400.** The transducer **102** receives a plurality of reflected waves due to reflection of the plurality of ultrasonic waves on the plurality of aquatic animals and generates a corresponding plurality of echo signals accordingly. The transceiver module **104** outputs a plurality of electric signals to the transducer **102** via the electric cable based on which the transducer **102** transmits the plurality of ultrasonic waves. Further, the transceiver module **104** acquires via the electric cable the plurality of echo signals, generated by the transducer **102** based on the plurality of reflected waves. Further, the transceiver module **104** may be configured to amplify, filter, and convert the corresponding plurality of analog echo signals into a plurality of digital reception signals. The tracking module **108** tracks with respect to time the plurality of aquatic animals, such as the fish **400,** among the underwater targets from the plurality of echo signals resulting from the corresponding plurality of ultrasonic waves.

The ultrasound incidence angle calculation module **110** calculates a plurality of incidence angles for the plurality of aquatic animals, which means that for each aquatic animal, a plurality of incidence angles are calculated across a plurality of a pings/transmissions, in a manner similar to the calculation of the incidence angles for the fish **400** as described in **FIG. 1.** It will be understood by a person skilled in the art the ultrasound incidence angle calculation module **110** may calculate the plurality of incidence angles using any one definition of the aforementioned definitions of the incidence angle described with reference to **FIGs.4A, 4B** and **4C.**

The parts distance calculation module **112** calculates a plurality of internal distances for a corresponding plurality of incidence angles of the ultrasonic waves on each aquatic animal of the plurality of aquatic animals. Thus, the parts distance calculation module **112** calculates a plurality of internal distances for each aquatic animal across the plurality of transmissions/pings.

As explained before, the parts distance calculation module **112** may calculate the internal distance between any two internal parts of each aquatic animal, such as the back and belly, the back and swimbladder, and the belly and swimbladder, for each incidence angle in a similar manner as described in **FIG. 1.** For the sake of ongoing discussion, it is assumed that the internal distance is the distance between the back and the swimbladder of the fish, and may be hereinafter also referred to as swimbladder-back distance.

In the context of the present disclosure, the transducer **102** transmits the first and second ultrasonic waves (*i.e.,* the first and second transmissions $T_n$ and $T_{n-1}$) toward a plurality of aquatic animals and generate corresponding first and second echo signals based on the reflection of the first and second ultrasonic waves from the plurality of aquatic animals. Further, for each aquatic animal, the ultrasound incidence angle calculation module **110** calculates first and second incidence angles $\theta_1$, and $\theta_2$ and the parts distance calculation module **112** calculates first and second internal distances $d_1$ and $d_2$ corresponding to first and second transmissions $T_n$ and $T_{n-1}$.

Further, for each aquatic animal, the internal structure information module **114** stores an incidence angle of the plurality of incidence angles and a corresponding internal distance of the plurality of internal distances as a pair. Thus, for each aquatic animal, the internal structure information module **114** stores a plurality of such pairs corresponding to the plurality of transmissions/pings. The calculation of pairs of incidence angles and internal distances for two tracked fishes are illustrated with reference to tables below:

**Table III (a)**

| Tracked fish 1 | |
|---|---|
| Incidence angle | Internal distance |
| $\theta_{11}$ | $d_{11}$ |
| $\theta_{12}$ | $d_{12}$ |
| $\theta_{13}$ | $d_{13}$ |

**Table III (b)**

| Tracked fish 2 | |
|---|---|
| Incidence angle | Internal distance |
| $\theta_{21}$ | $d_{21}$ |
| $\theta_{22}$ | $d22$ |

In Table III(a), $\theta_{11},$ $\theta_{12},$ and $\theta_{13}$ are first, second and third incidence angles and $d_{11}$, $d_{12}$, and $d_{13}$ are first, second,

and third internal distances of the first tracked fish, across the first, second, and third transmissions (pings) of the ultrasonic waves on the first tracked fish, respectively. Similarly, in Table III(b), $\theta_{21}$ and $\theta_{22}$ are first and second incidence angles and $d_{21}$ and $d_{22}$ are first and second internal distances of the second tracked fish, across the first and second transmissions (pings) of the ultrasonic waves on the second tracked fish, respectively.

Further, for each aquatic animal, the internal structure information module **114** stores an internal structure based on corresponding plurality of pairs in a manner similar to the storage of the internal structure of the fish **400**.

The tracking module **108,** the ultrasound incidence angle calculation module **110,** and the parts distance calculation module **112,** repeatedly perform respective calculations for a large number of transmit/receive cycles (pings) to obtain internal distances (for example, swimbladder-back distance) and incidence angles for a large number of tracked fishes. In one embodiment, the internal distances calculated for the large number of pings for corresponding incidence angles are stored in the internal structure information module **114** as internal distance data and incidence angle data for the plurality of aquatic animals.

The histogram calculation module **702** divides the entire range of values of the internal distance data and the entire range of values of incidence angle data into a series of intervals and then the number of values (occurrence) that fall into each interval is counted as shown in table below:

**Table IV**

|  | $d_1$-$d_2$ | $d_2$-$d_3$ | ... | $d_{i-1}$-$d_i$ |
|---|---|---|---|---|
| $\theta_1$-$\theta_2$ | $Count_{1,1}$ | $Count_{1,2}$ | ... | $Count_{1,i-1}$ |
| $\theta_2$-$\theta_3$ | $Count_{2,1}$ | $Count_{2,2}$ | ... | $Count_{2,i-1}$ |
|  | ... | ... | ... | ... |
| $\theta_{j-1}$-$\theta_j$ | $Count_{j-1,1}$ | $Count_{j-1,2}$ | ... | $Count_{j-1,i-1}$ |

In Table IV, $\theta_1$ - $\theta_2$, $\theta_2$ - $\theta_3$, ..., $\theta_{j-1}$ - $\theta_j$ are the series of intervals for the incidence angle data and $d_1$ - $d_2$, $d_2$ - $d_3$, ..., $d_{i-1}$ - $d_i$ are the series of intervals for the internal distance data of the plurality of tracked fish. In one example, each interval for the incidence angle data corresponds to 1° such that the series of intervals for the incidence angle data are -1° to 0°, 0° to 1°, and so on, and each interval for the internal distance data corresponds to 0.001 meters (m) such that the series of intervals for the internal distance data are 0m to - 0.001m, -0.001m to -0.002m, and so on. Further, a count for each interval of the internal distance data and corresponding interval incidence angle data is stored in Table IV. Thus, the Table IV corresponds to a global internal structure of the plurality of aquatic animals. In an embodiment, the internal structure information module **114** stores the global internal structure of the plurality of fish, for example, in the form of Table IV.

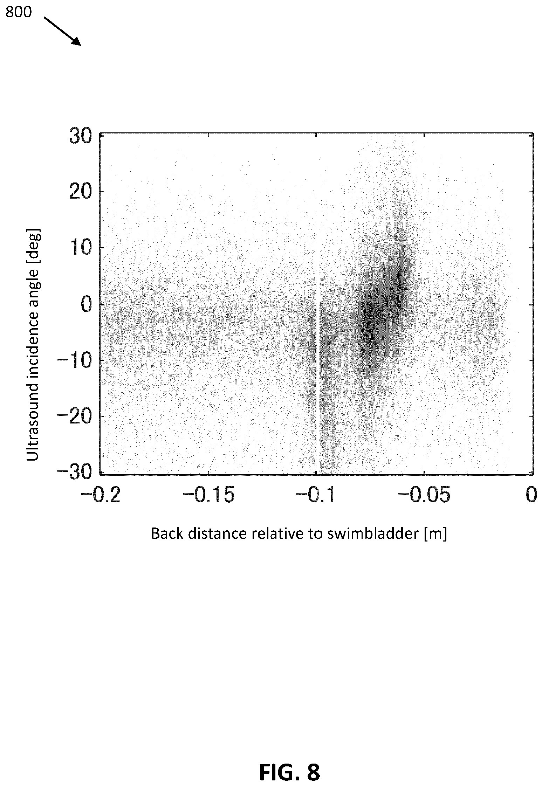

Referring to **FIGs. 7** and **8,** the histogram calculation module **702** generates a histogram **800** showing a distribution of incidence angle and internal distance of the plurality of aquatic animals from the internal structure of the plurality of aquatic animals, *i.e.,* based on the Table IV. In another embodiment, the internal structure information module **114** stores the global internal structure of the plurality of aquatic animals, for example, in the form of the histogram **800**. The histogram **800** is graphical representation of the global internal structure of the plurality of fish represented by the Table IV.

The histogram **800** represents the number of values (count) for the series of intervals of incidence angle data and internal distance data shown in **Table IV.** In an example, the histogram **800** has been obtained by tracking 29672 yellowtail fishes raised in the fish cage **200**.

The densely populated areas of the histogram **800** show combinations of (incidence angle, internal distance) that most often occur. As described above, the transducer **102** is installed so as to send the transmitted wave downward from near the water surface. Therefore, a negative value on the horizontal axis of the histogram **800** means the back side of the fish from the position of the swimbladder (reference position), and a positive value means the ventral side of the fish from the position of the swimbladder.

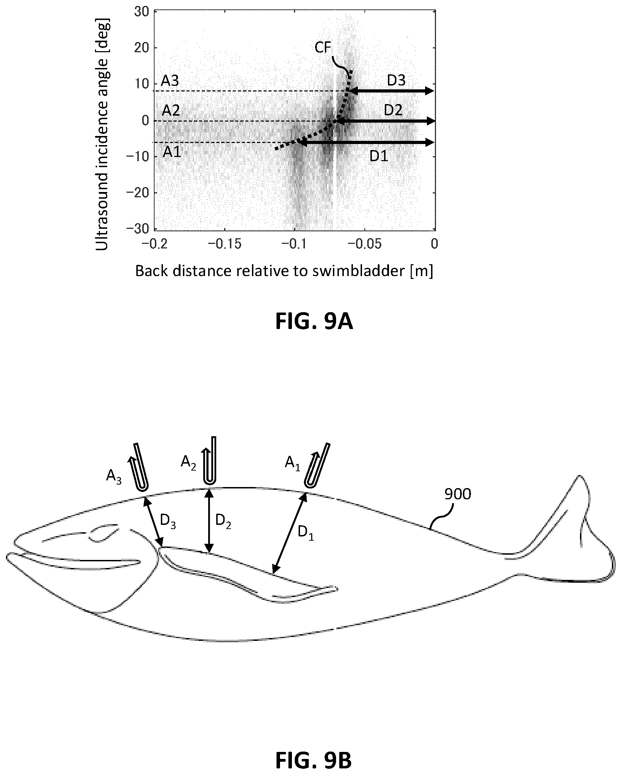

FIG. 9A illustrates a relative position of the swimbladder and the back of fish swimming in the fish cage **200** obtained from the histogram **800**. For example, when a curve (CF) is fitted to the trend of the histogram **800** *(i.e.,* densely populated areas of the histogram **800),** the mean weight calculation module **904** extracts the internal distances $D_1$, $D_2$, $D_3$ from the fitted curve (CF) to the reference position (swimbladder) from the histogram **800** at the corresponding incidence angles $A_1$, $A_2$, $A_3$, respectively. The internal distances $D_1$, $D_2$, and $D_3$ indicate the global internal distances of the fish in the fish cage **200** at the ultrasound incidence angles $A_1$, $A_2$, and $A_3$, respectively. It will be apparent to a person skilled in the art that the fitted curve (CF) may be fitted manually or automatically using a conventional curve fitting algorithm.

FIG. 9B illustrates a global internal structure for the plurality of aquatic animals, such as a fish **900,** and the global internal distances $D_1$, $D_2$, and $D_3$ obtained at different incidence angles $A_1$, $A_2$, and $A_3$. The internal structure information module **114** determines a correspondence between the global internal distances and the different incidence angles, and stores the correspondence between the global internal distances and the different incidence angles as the global internal structure of the plurality of aquatic animals, for example, in the form of the table IV or the histogram **800.**

In the present embodiment, the back-swimbladder distance at a given incidence angle is measured using the fitted curve (CF). In various other embodiments, at a given incidence angle, a distance between a region on the histogram **800** that corresponds to the maximum count and the reference position (swimbladder) may be used instead to measure the back-swimbladder distance.

Referring back to **FIG.7,** the mean weight calculation module **704** calculates a mean weight of the plurality of tracked fish based on a multiplication of the plurality of internal distances (corresponding to the histogram **800),** each raised to the power of a predetermined value. The calculation of the mean weight is represented as follows:

$$Wmean = \gamma_0 \prod_1^M D_m^{\gamma_m}$$

where distances $D_m$ $(1 \le m \le M)$ between the fitted curve CF and the reference position (swimbladder in the example histogram above) are calculated at given ultrasound incidence angles $A_m$, and $\gamma_m$ $(0 \le m \le M)$ are predetermined values based on experiment data for a given fish species, such as the yellowtail fish.

Although not shown, the signal processing module **106** may generate display data for displaying data relating to the internal structure and weight of the fish. Further, the signal processing module **106** may output the display data to an appropriate display device (not shown) connected to the signal processing module **106.**

In the present embodiment, the weight of the individual fish **400** and the mean weight of all the fish in the fish cage **200** are measured based on the internal structure of the individual fish **400** and the global internal structure of all the fish. In various other embodiments, a size of the individual fish **400** and a mean size of all the fish in the fish cage **200** may be measured based on the internal structure of the individual fish **400** and the global internal structure of all the fish. The size of a fish may include at least one of a fish length, a fish height, a fish breadth, or the like.

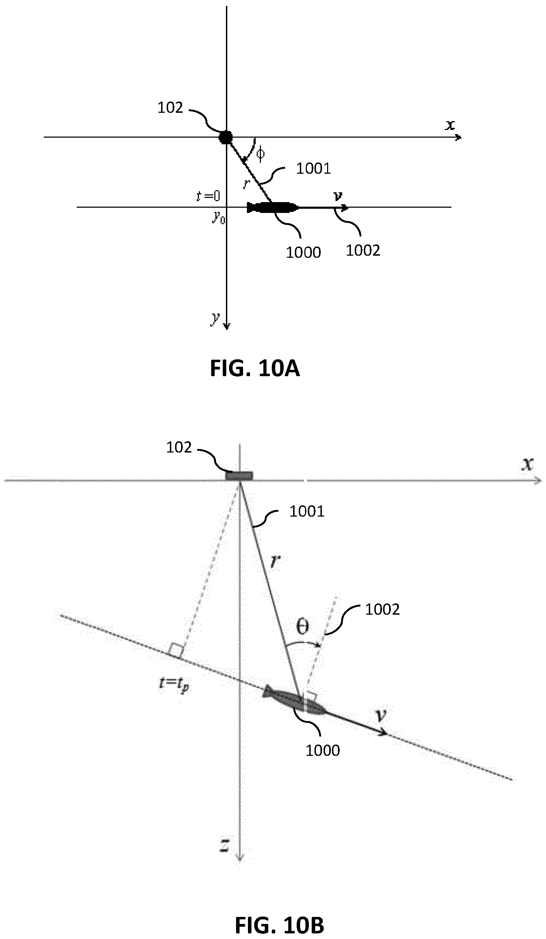

**FIG. 10A** illustrates a top view showing the calculation of the incidence angle θ when the transducer **102** includes a single receiving channel, in accordance with an embodiment of the present disclosure. **FIG. 10B** illustrates a side view showing the calculation of the incidence angle θ when the transducer **102** includes a single receiving channel, in accordance with an embodiment of the present disclosure.

As shown in **FIGs. 10A** and **10B,** a fish **1000** swims linearly below a single beam transducer **102** *(i.e.,* the transducer **102** that includes a single receiving channel) placed at a fixed position in the fish cage **200** and oriented vertically down. A 3D cartesian coordinate system is shown in the **FIGs. 10A** and **10B,** such that the transducer **102** is placed at a center of the 3D cartesian coordinate system. The fish **1000** is at a distance **r** from the transducer **102** and swims at a speed **v.**

At time t=0, the fish **1000** crosses the YZ plane. The distance **r** varies with time **t** according to the following equation (hyperbolic curve):

$$r^2 = v^2(t - t_p)^2 + r_p{}^2 \dots\dots\dots\dots.(4)$$

where **$t_p$** corresponds to the time **t** when the distance **r** is minimum, and **$r_p$** is the minimum distance between the fish **1000** and the transducer **102.**

As shown in **FIG. 10B,** the incidence angle θ is an angle between a fish-transducer vector **1001** *(i.e.,* a first line that connects the fish **1000** and the transducer **102)** and a perpendicular vector **1002** *(i.e.,* a second line that is perpendicular to a direction in which the fish **1000** is swimming). The incidence angle θ at time **t** may be expressed using the following equation:

$$\theta = -\sin^{-1} \frac{v(t - t_p)}{r} \dots\dots\dots\dots\dots.(5)$$

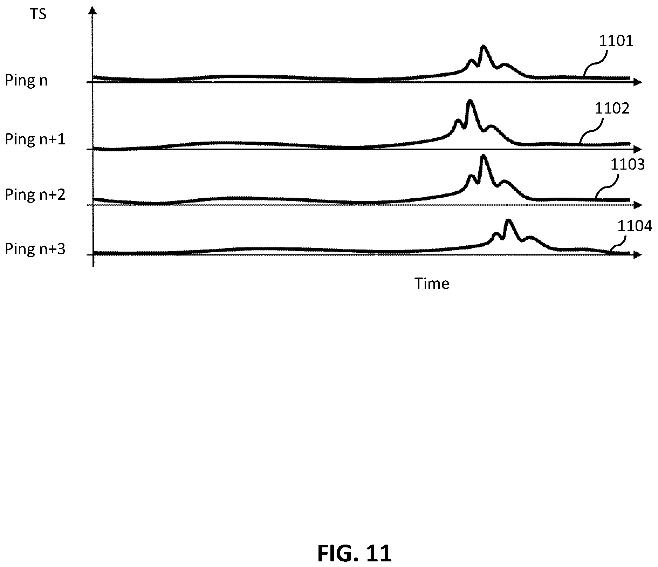

**FIG. 11** illustrates various echo signals, *i.e.,* echo signals **1101, 1102, 1103,** and **1104,** generated at different time instances *i.e.,* during pings **n, n+1, n+2,** and **n+3,** respectively, when the fish **1000** swims below the single receiving channel transducer **102.**

During each ping, a corresponding distance from the fish **1000** to the transducer **102** may be calculated by measuring a time taken by the ultrasonic wave to reflect from the swimbladder of the fish **1000,** by multiplying that time with the speed of sound in water, and by dividing the multiplication result by two. The distances r for each ping may be illustrated as below:

**Table V**

| Ping | Distance to fish |
|------|------------------|
| Ping n | $r_n$ |
| Ping n+1 | $r_{n-1}$ |
| Ping n+2 | $r_{n-2}$ |
| Ping n+3 | $r_{n-3}$ |

Referring back to **FIG.10B,** it is assumed that the distance between the fish **1000** and the transducer **102** changes with respect to time according to the below equation of a hyperbolic curve:

$$r^2 = at^2 + bt + c \,...\,(6)$$

where r is the distance and t is time.

The parameters a, b, and c of the equation (6) are obtained using, for example, least squares method. Based on the equations (4) and (6), the speed of the fish v and the time $t_p$ are calculated as follows:

$$v = \sqrt{a}$$

$$t_p = -\frac{b}{2a}$$

Herein, the ultrasound incidence angle calculation module **110** calculates the incidence angle θ with equation (5) at each ping by substituting the value of the speed **v** of the fish **1000,** and the time $t_p$. In the context of the present disclosure, when the transducer **102** generates the first and second echo signals **1101** and **1102** based on the first and second ultrasonic waves *(i.e.,* the first and second pings **n** and **n+1**), the first and second distances $r_n$ and $r_{n+1}$ are calculated as described above, respectively. In the embodiment, the second ultrasonic wave is transmitted after the first ultrasonic wave. Thus, at the second time instance, the ultrasound incidence angle calculation module **110** calculates a second incidence angle $\theta_{n+1}$ based on the first distance $r_n$ between the aquatic animal *(i.e.,* the fish **1000)** and the transducer **102** in the first echo signal **1101** and the second distance $r_{n+1}$ between the aquatic animal and the transducer **102** in the second echo signal **1102.** The ultrasound incidence angle calculation module **110** calculates the second incidence angle $\theta_{n+1}$, based on an assumption that the first and second distances $r_n$ and $r_{n+1}$ change with time based on a hyperbolic curve, using the equation (6).

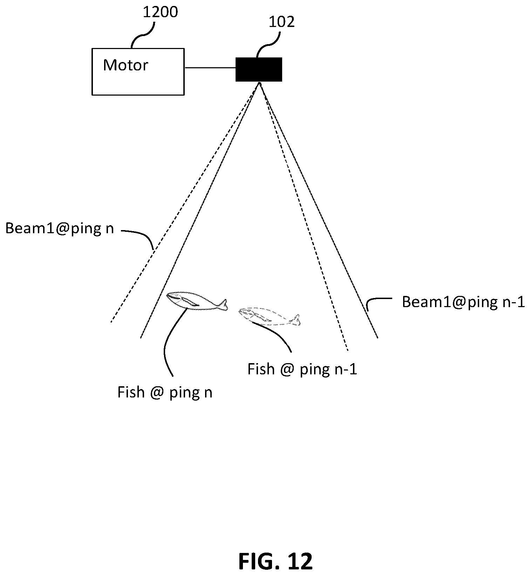

In the aforementioned embodiments, the transducer **102** is fixed at one position and transmits the ultrasonic waves in a fixed direction, *i.e.,* from above towards the aquatic animals. In various other embodiments, the transducer **102** may be coupled to a motor **1200** such that the transducer **102,** transmits the first and second ultrasonic waves towards the aquatic animals in different directions at two different time instances (pings) as shown in **FIG. 12.**

As shown in **FIG. 12,** the motor **1200** is coupled to the transducer **102,** and configured to move (e.g., rotate) the transducer **102** to change a direction in which the transducer **102** transmits the first and second ultrasonic waves. The transducer **102** transmits the first ultrasonic wave in a first direction at a first time instance (first ping n-**1),** and the second ultrasonic wave in a second direction different from the first direction at a second time instance (second ping **n).**

In the present embodiment, the single transducer **102** is used to transmit ultrasonic waves towards the aquatic animals and receive the reflected waves. In various other embodiments, more than one transducer may be used to transmit ultrasonic waves towards the aquatic animals and receive the reflected waves.

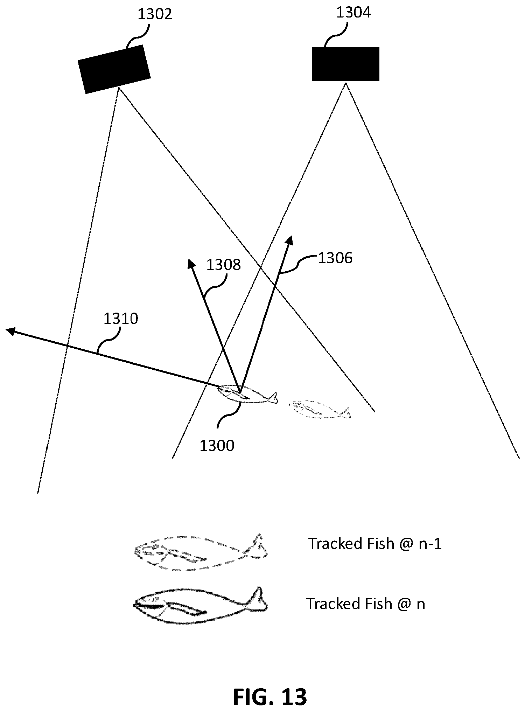

FIG. 13 illustrates tracking of a fish **1300** using a transducer having first and second transducer elements **1302** and **1304.** The first and second transducer elements **1302** and **1304** are used to track an identical fish on a plurality of pings based on which the ultrasound incidence angle and the internal distance are calculated at each position of the tracked fish **1300.** The first and second transducer elements **1302** and **1304** are placed at different positions, *i.e.,* the first and second transducer elements **1302** and **1304** are spaced apart by a predetermined distance, and may be facing toward different directions. Thus, the first and second transducer elements **1302** and **1304** transmit the first and second ultrasonic waves in directions that are different with respect to each other, respectively.

The first and second transducer elements **1302** and **1304** may transmit the first and second ultrasonic waves, respectively, at the same time with different frequencies so that the reception waves can be differentiated from each other. Based on the reflected waves, the first and second transducer elements **1302** and **1304** detect the fish **1300.** To determine, if the first and second transducer elements **1302** and **1304** detect the same fish **1300,** a 3D position of the fish detected by the first transducer element **1302** is calculated with reference to a position of the first transducer element **1302,** and a 3D position of the fish detected by the second transducer element **1304** is calculated with reference to a position of the second transducer element **1304.** As a relative position of the first and second transducer elements **1302** and **1304,** *i.e.,* the predetermined distance between the first and second transducer elements **1302** and **1304,** is known in advance, it is therefore possible to determine if the detected fish by the first and second transducer elements **1302** and **1304** are at the same position and therefore if they are actually the same fish, *i.e.,* the fish **1300.**

Further, the first and second fish-transducer vectors **1306** and **1308** and a swimming direction vector **1310** are obtained for the tracked fish **1300.** Thus, based on the first and second fish-transducer vectors **1306** and **1308** and the swimming direction vector **1310,** two incidence angles corresponding to the first and second ultrasonic waves and corresponding internal parts distances may be obtained at the same time for the fish **1300.**

In the above embodiments, the transducer **102** is mainly arranged to transmit an ultrasonic beam of a given beam width vertically downward, from above the fish **1300.** However, there is no limitation about the position of the transducer **102** relative to the fish **1300** and the orientation of the beam. The transducer **102** may be placed at any position and the beam can be oriented in any direction as long as at least a part of the fish is within the beam.

In the above embodiments, the individual fish weight is measured after calculating the internal distances at a plurality of pings. However, there is no such limitation. The individual fish weight may be for example calculated after calculating the internal distance $d_1$ at an incidence angle $\theta_1$ using the following formula:

$$Weight = \beta_0 * d_1^{\beta_1}$$

where $\beta_0$ and $\beta_1$ are predetermined values for a particular fish species.

Further, the width of the beam of ultrasonic wave transmitted by the transducer 102 may be adjusted depending on a depth of the fish or the swimming speed of the fish, to improve detection of the fish. In one embodiment, the width of the beam is inversely proportional to the depth of the fish. The variation of the beam width with respect to the variation of the depth of the fish may be illustrated as below:

**Table VI**

| Depth [m] | Beam width [deg] |
|-----------|------------------|
| 1         | 24               |
| 3         | 8                |
| 6         | 4                |

Further, in one embodiment, the width of the beam is directly proportional to the speed of the fish. The variation of the beam width with respect to the variation of the speed of the fish may be illustrated as below:

**Table VII**

| Speed [kt] | Beam width [deg] |
|------------|------------------|
| 1          | 8                |
| 2          | 16               |

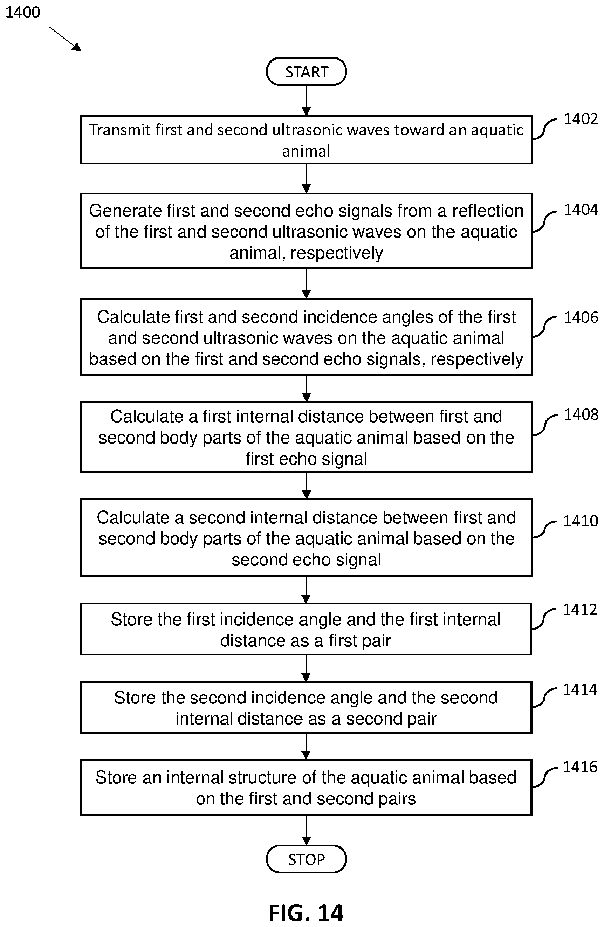

**FIG. 14** is a flow chart illustrating an aquatic animal shape calculation method **1400,** in accordance with an embodiment of the present disclosure. Various steps of the aquatic animal shape calculation method **1400** have been explained with reference to **FIG. 1.**

At step **1402,** the transducer **102** transmits first and second ultrasonic waves toward an aquatic animal. At step **1404,** the transducer **102** generates first and second echo signals from a reflection of the first and second ultrasonic waves on the aquatic animal, respectively.

At step **1406,** the ultrasound incidence angle calculation module **110** calculates first and second incidence angles of the first and second ultrasonic waves on the aquatic animal based on the first and second echo signals, respectively.

At step **1408,** the parts distance calculation module **112** calculates a first internal distance between first and second body parts of the aquatic animal based on the first echo signal. At step **1410,** the parts distance calculation module **112** calculates a second internal distance between first and second body parts of the aquatic animal, based on the second echo signal.

At step **1412,** the internal structure information module **114** stores the first incidence angle and the first internal distance as a first pair. At step **1414,** the internal structure information module **114** stores the second incidence angle and the second internal distance as a second pair. At step **1416,** the internal structure information module **114** stores an internal structure of the aquatic animal based on the first and second pairs.

It is to be understood that not necessarily all objectives or advantages may be achieved in accordance with any particular embodiment described herein. Thus, for example, those skilled in the art will appreciate that certain embodiments may be configured to operate in a manner that achieves or optimizes one advantage or group of advantages as taught herein without necessarily achieving other objects or advantages as may be taught or suggested herein.

**Claims**

1. A device (100, 700) for calculating an internal structure of an aquatic animal, the device comprising:

   a transducer (102) configured to transmit first and second ultrasonic waves toward an aquatic animal and generate first and second echo signals from a reflection of the first and second ultrasonic waves on the aquatic animal, respectively;
   an ultrasound incidence angle calculation module (110) configured to calculate first and second incidence angles of the first and second ultrasonic waves on the aquatic animal based on the first and second echo signals, respectively;
   a parts distance calculation module (112) configured to:

      calculate a first internal distance between first and second body parts of the aquatic animal based on the first echo signal; and
      calculate a second internal distance between first and second body parts of the aquatic animal based on the second echo signal; and

   an internal structure information module (114) configured to:

      store the first incidence angle and the first internal distance as a first pair;
      store the second incidence angle and the second internal distance as a second pair; and
      store the internal structure of the aquatic animal, wherein the internal structure is based on the first and second pairs;
      wherein:

         the second ultrasonic wave is transmitted after the first ultrasonic wave;
         the ultrasound incidence angle calculation module (110) calculates the second incidence angle based on a first distance between the aquatic animal and the transducer (102) in the first echo signal and a second distance between the aquatic animal and the transducer (102) in the second echo signal; and
         the ultrasound incidence angle calculation module (110) calculates the second incidence angle based on an assumption that the first and second distances change with time based on a hyperbolic curve;
         the ultrasound incidence angle calculation module (110) calculates the first incidence angle based on another distance between the aquatic animal and the transducer (102) in another echo signal and the first distance between the aquatic animal and the transducer (102) in the first echo signal, the other echo signal being generated from a reflection of another ultrasonic wave, transmitted before the first ultrasonic wave, on the aquatic animal; and
         the ultrasonic incidence angle calculation module (110) calculates the first incidence angle based on an assumption that the other distance and the first distance change with time based on a hyperbolic curve.

2. The device (100) according to claim 1, further comprising:
   an individual weight calculation module (116) configured to calculate a weight of the aquatic animal, based on the internal structure of the aquatic animal, by multiplying the first internal distance raised to the power of a first predetermined value, with the second internal distance raised to the power of a second predetermined value.

3. The device (700) according to claim 1 or claim 2, wherein:

   the transducer (102) is configured to transmit the first and second ultrasonic waves toward a plurality of aquatic animals including the aquatic animal;

the ultrasound incidence angle calculation module (110) is configured to calculate the first and second incidence angles for the plurality of aquatic animals;

the parts distance calculation module (112) is configured to calculate the first and second internal distances for the plurality of aquatic animals; and

the internal structure information module (114) is configured to store the internal structure of the plurality of aquatic animals.

4. The device (700) of claim 3, further comprising:

a histogram calculation module (702) configured to calculate a histogram representing a distribution of incidence angle and internal distance of the plurality of aquatic animals from the internal structure of the plurality of aquatic animals, wherein the internal structure information module (114) is further configured to store the histogram as a global internal structure of the plurality of aquatic animals.

5. The device (700) of claim 4, further comprising:

a mean weight calculation module (704) configured to calculate a mean weight of the plurality of aquatic animals from the histogram by extracting from the histogram a plurality of internal distances at a corresponding plurality of incidence angles, and by calculating the mean weight based on the extracted plurality of internal distances.

6. The device (700) of claim 5, wherein the mean weight calculation module (704) calculates the mean weight based on a multiplication of the plurality of internal distances, each raised to the power of a predetermined value.

7. The device (100, 700) according to any one of claims 1 to 6, further comprising:

a motor (1200) coupled to the transducer (102), and configured to move the transducer (102) to change a direction in which the transducer (102) transmits the first and second ultrasonic waves, wherein the transducer (102) transmits the first ultrasonic wave in a first direction, and the second ultrasonic wave in a second direction different from the first direction.

8. The device (100, 700) of claim 1, wherein the transducer (102) comprises first and second transducer elements (1302 and 1304) spaced apart by a predetermined distance, and wherein the first transducer element (1302) transmits the first ultrasonic wave and the second transducer element (1304) transmits the second ultrasonic wave.

9. The device (100, 700) of claim 8, wherein the first and second transducer elements (1302 and 1304) transmit the first and second ultrasonic waves in directions that are different with respect to each other, respectively.

10. A method for calculating an internal structure of an aquatic animal, the method comprising:

transmitting first and second ultrasonic waves toward an aquatic animal;

generating first and second echo signals from a reflection of the first and second ultrasonic waves on the aquatic animal, respectively;

calculating first and second incidence angles of the first and second ultrasonic waves on the aquatic animal based on the first and second echo signals, respectively;

calculating a first internal distance between first and second body parts of the aquatic animal based on the first echo signal;

calculating a second internal distance between first and second body parts of the aquatic animal based on the second echo signal;

storing the first incidence angle and the first internal distance as a first pair;

storing the second incidence angle and the second internal distance as a second pair; and

storing the internal structure of the aquatic animal, wherein the internal structure is based on the first and second pairs;

wherein the second ultrasonic wave is transmitted after the first ultrasonic wave;

and wherein the method further comprises calculating the second incidence angle based on a first distance between the aquatic animal and a transducer (102) in the first echo signal and a second distance between the aquatic animal and the transducer (102) in the second echo signal; and

calculating the second incidence angle based on an assumption that the first and second distances change with time based on a hyperbolic curve;

and wherein the first incidence angle is calculated based on another distance between the aquatic animal and the transducer (102) in another echo signal and the first distance between the aquatic animal and the transducer (102) in the first echo signal, the other echo signal being generated from a reflection of another ultrasonic wave, transmitted before the first ultrasonic wave, on the aquatic animal; and

the first incidence angle is calculated based on an assumption that the other distance and the first distance change with time based on a hyperbolic curve.

**11.** A non-transitory computer readable medium having stored thereon computer-executable instructions which, when executed by the device of claim 1, cause the device to execute the method of claim 10.

**Patentansprüche**

**1.** Gerät (100, 700) zur Berechnung der Innenstruktur eines Wassertieres, wobei das Gerät Folgendes umfasst:

einen Wandler (102), konfiguriert zum Übertragen einer ersten und einer zweiten Ultraschallwelle in Richtung eines Wassertieres und zum Erzeugen eines ersten und eines zweiten Echosignals aus einer Reflexion der ersten bzw. zweiten Ultraschallwelle an dem Wassertier;
ein Ultraschalleinfallswinkel-Berechnungsmodul (110), konfiguriert zum Berechnen eines ersten und zweiten Einfallswinkels der ersten bzw. zweiten Ultraschallwelle an dem Wassertier auf der Basis des ersten bzw. zweiten Echosignals;
ein Teileabstandsberechnungsmodul (112), konfiguriert zum:

Berechnen eines ersten inneren Abstands zwischen ersten und zweiten Körperteilen des Wassertiers auf der Basis des ersten Echosignals; und
Berechnen eines zweiten inneren Abstands zwischen ersten und zweiten Körperteilen des Wassertiers auf der Basis des zweiten Echosignals; und

ein Innenstrukturinformationsmodul (114), konfiguriert zum:

Speichern des ersten Einfallswinkels und des ersten inneren Abstands als erstes Paar;
Speichern des zweiten Einfallswinkels und des zweiten inneren Abstands als zweites Paar; und
Speichern der Innenstruktur des Wassertiers, wobei die Innenstruktur auf dem ersten und dem zweiten Paar basiert;
wobei:

die zweite Ultraschallwelle nach der ersten Ultraschallwelle übertragen wird;
das Ultraschalleinfallswinkel-Berechnungsmodul (110) den zweiten Einfallswinkel auf der Basis eines ersten Abstands zwischen dem Wassertier und dem Wandler (102) im ersten Echosignal und eines zweiten Abstands zwischen dem Wassertier und dem Wandler (102) im zweiten Echosignal berechnet; und
das Ultraschalleinfallswinkel-Berechnungsmodul (110) den zweiten Einfallswinkel auf der Basis einer Annahme berechnet, dass sich der erste und der zweite Abstand mit der Zeit auf der Basis einer hyperbolischen Kurve ändern;
das Ultraschalleinfallswinkel-Berechnungsmodul (110) den ersten Einfallswinkel auf der Basis eines weiteren Abstands zwischen dem Wassertier und dem Wandler (102) in einem weiteren Echosignal und des ersten Abstands zwischen dem Wassertier und dem Wandler (102) im ersten Echosignal berechnet, wobei das weitere Echosignal aus einer Reflexion einer vor der ersten Ultraschallwelle übertragenen weiteren Ultraschallwelle an dem Wassertier erzeugt wird; und
das Ultraschalleinfallswinkel-Berechnungsmodul (110) den ersten Einfallswinkel auf der Basis einer Annahme berechnet, dass sich der weitere Abstand und der erste Abstand mit der Zeit auf der Basis einer hyperbolischen Kurve ändern.

**2.** Gerät (100) nach Anspruch 1, das ferner Folgendes umfasst:
ein Einzelgewichtberechnungsmodul (116), konfiguriert zum Berechnen eines Gewichts des Wassertiers auf der Basis der Innenstruktur des Wassertiers durch Multiplizieren des ersten inneren Abstands, potenziert mit einem ersten vorbestimmten Wert, mit dem zweiten inneren Abstand, potenziert mit einem zweiten vorbestimmten Wert.

**3.** Gerät (700) nach Anspruch 1 oder Anspruch 2, wobei:

der Wandler (102) zum Übertragen der ersten und zweiten Ultraschallwelle in Richtung mehrerer Wassertiere, einschließlich des Wassertiers, konfiguriert ist;

das Ultraschalleinfallswinkel-Berechnungsmodul (110) zum Berechnen des ersten und zweiten Einfallswinkels für die mehreren Wassertiere konfiguriert ist;

das Teileabstandsberechnungsmodul (112) zum Berechnen des ersten und zweiten inneren Abstands für die mehreren Wassertiere konfiguriert ist; und

das Innenstrukturinformationsmodul (114) zum Speichern der Innenstruktur der mehreren Wassertiere konfiguriert ist.

4. Gerät (700) nach Anspruch 3, das ferner Folgendes umfasst:
ein Histogrammberechnungsmodul (702), konfiguriert zum Berechnen eines Histogramms, das eine Verteilung von Einfallswinkel und innerem Abstand der mehreren Wassertiere anhand der Innenstruktur der mehreren Wassertiere darstellt, wobei das Innenstrukturinformationsmodul (114) ferner zum Speichern des Histogramms als globale Innenstruktur der mehreren Wassertiere konfiguriert ist.

5. Gerät (700) nach Anspruch 4, das ferner Folgendes umfasst:
ein Durchschnittsgewichtsberechnungsmodul (704), konfiguriert zum Berechnen eines Durchschnittsgewichts der mehreren Wassertiere aus dem Histogramm durch Extrahieren mehrerer innerer Abstände bei einer entsprechenden mehreren Einfallswinkeln aus dem Histogramm und durch Berechnen des Durchschnittsgewichts auf der Basis der extrahierten mehreren inneren Abstände.

6. Gerät (700) nach Anspruch 5, wobei das Durchschnittsgewichtsberechnungsmodul (704) das Durchschnittsgewicht auf der Basis einer Multiplikation der mehreren inneren Abstände, jeweils potenziert mit einem vorbestimmten Wert, berechnet.

7. Gerät (100, 700) nach einem der Ansprüche 1 bis 6, das ferner Folgendes umfasst:
einen mit dem Wandler (102) gekoppelten Motor (1200), konfiguriert zum Bewegen des Wandlers (102), um eine Richtung zu ändern, in der der Wandler (102) die erste und zweite Ultraschallwelle überträgt, wobei der Wandler (102) die erste Ultraschallwelle in einer ersten Richtung und die zweite Ultraschallwelle in einer sich von der ersten Richtung unterscheidenden zweiten Richtung überträgt.

8. Gerät (100, 700) nach Anspruch 1, wobei der Wandler (102) ein erstes und zweites Wandlerelement (1302 und 1304) umfasst, die in einem vorbestimmten Abstand voneinander angeordnet sind, und wobei das erste Wandlerelement (1302) die erste Ultraschallwelle überträgt und das zweite Wandlerelement (1304) die zweite Ultraschallwelle überträgt.

9. Gerät (100, 700) nach Anspruch 8, wobei das erste und zweite Wandlerelement (1302 und 1304) die erste und zweite Ultraschallwelle in Richtungen übertragen, die sich jeweils voneinander unterscheiden.

10. Verfahren zum Berechnen einer Innenstruktur eines Wassertiers, wobei das Verfahren Folgendes beinhaltet:

Übertragen einer ersten und einer zweiten Ultraschallwelle in Richtung eines Wassertiers;
Erzeugen eines ersten und eines zweiten Echosignals aus einer Reflexion der ersten bzw. zweiten Ultraschallwelle an dem Wassertier;
Berechnen eines ersten und zweiten Einfallswinkels der ersten bzw. zweiten Ultraschallwelle an dem Wassertier auf der Basis des ersten bzw. zweiten Echosignals;
Berechnen eines ersten inneren Abstands zwischen ersten und zweiten Körperteilen des Wassertiers auf der Basis des ersten Echosignals;
Berechnen eines zweiten inneren Abstands zwischen ersten und zweiten Körperteilen des Wassertiers auf der Basis des zweiten Echosignals;
Speichern des ersten Einfallswinkels und des ersten inneren Abstands als erstes Paar;
Speichern des zweiten Einfallswinkels und des zweiten inneren Abstands als zweites Paar; und
Speichern der Innenstruktur des Wassertiers, wobei die Innenstruktur auf dem ersten und zweiten Paar basiert;
wobei die zweite Ultraschallwelle nach der ersten Ultraschallwelle übertragen wird;
und wobei das Verfahren ferner Folgendes beinhaltet:

Berechnen des zweiten Einfallswinkels auf der Basis eines ersten Abstands zwischen dem Wassertier und einem Wandler (102) im ersten Echosignal und eines zweiten Abstands zwischen dem Wassertier und dem Wandler (102) im zweiten Echosignal; und

Berechnen des zweiten Einfallswinkels auf der Basis einer Annahme, dass sich der erste und zweite Abstand mit der Zeit auf der Basis einer hyperbolischen Kurve ändern;

und wobei der erste Einfallswinkel auf der Basis eines weiteren Abstands zwischen dem Wassertier und dem Wandler (102) in einem weiteren Echosignal und des ersten Abstands zwischen dem Wassertier und dem Wandler (102) im ersten Echosignal berechnet wird, wobei das weitere Echosignal aus einer Reflexion einer vor der ersten Ultraschallwelle übertragenen weiteren Ultraschallwelle an dem Wassertier erzeugt wird; und der erste Einfallswinkel auf der Basis einer Annahme berechnet wird, dass sich der weitere Abstand und der erste Abstand mit der Zeit auf der Basis einer hyperbolischen Kurve ändern.

11. Nicht-transitorisches, computerlesbares Medium, auf dem computerausführbare Befehle gespeichert sind, die bei Ausführung durch das Gerät nach Anspruch 1 das Gerät zum Ausführen des Verfahrens nach Anspruch 10 veranlassen.

**Revendications**

1. Dispositif (100, 700) permettant de calculer une structure interne d'un animal aquatique, le dispositif comprenant :

un transducteur (102), configuré pour émettre des première et seconde ondes ultrasonores vers un animal aquatique et générer des premier et second signaux d'écho à partir, respectivement, d'une réflexion des première et seconde ondes ultrasonores sur l'animal aquatique ;
un module de calcul d'angle d'incidence d'ultrasons (110), configuré pour calculer des premier et second angles d'incidence des première et seconde ondes ultrasonores sur l'animal aquatique, sur la base, respectivement, des premier et second signaux d'écho ;
un module de calcul de distance entre parties (112), configuré pour :

calculer une première distance interne entre des première et seconde parties de corps de l'animal aquatique, sur la base du premier signal d'écho ; et
calculer une seconde distance interne entre des première et seconde parties de corps de l'animal aquatique, sur la base du second signal d'écho ; et

un module d'informations de structure interne (114), configuré pour :

stocker le premier angle d'incidence et la première distance interne sous la forme d'une première paire ;
stocker le second angle d'incidence et la seconde distance interne sous la forme d'une seconde paire ; et
stocker la structure interne de l'animal aquatique, la structure interne étant basée sur les première et seconde paires ;

la seconde onde ultrasonore étant émise après la première onde ultrasonore ;
le module de calcul d'angle d'incidence d'ultrasons (110) calculant le second angle d'incidence sur la base d'une première distance entre l'animal aquatique et le transducteur (102) dans le premier signal d'écho et d'une seconde distance entre l'animal aquatique et le transducteur (102) dans le second signal d'écho ; et
le module de calcul d'angle d'incidence d'ultrasons (110) calculant le second angle d'incidence sur la base d'une hypothèse que les première et seconde distances varient dans le temps sur la base d'une courbe hyperbolique ;
le module de calcul d'angle d'incidence d'ultrasons (110) calculant le premier angle d'incidence sur la base d'une autre distance entre l'animal aquatique et le transducteur (102) dans un autre signal d'écho et de la première distance entre l'animal aquatique et le transducteur (102) dans le premier signal d'écho, l'autre signal d'écho étant généré à partir d'une réflexion sur l'animal aquatique d'une autre onde ultrasonore, émise avant la première onde ultrasonore ; et
le module de calcul d'angle d'incidence d'ultrasons (110) calculant le premier angle d'incidence sur la base d'une hypothèse que l'autre distance et la première distance varient dans le temps sur la base d'une courbe hyperbolique.

2. Dispositif (100) selon la revendication 1, comprenant en outre :
un module de calcul de poids individuel (116), configuré pour calculer un poids de l'animal aquatique, sur la base de la structure interne de l'animal aquatique, en multipliant la première distance interne, élevée à la puissance d'une première valeur prédéterminée, par la seconde distance interne élevée à la puissance d'une seconde valeur prédéterminée.

3. Dispositif (700) selon la revendication 1 ou 2,

le transducteur (102) étant configuré pour émettre les première et seconde ondes ultrasonores vers une pluralité d'animaux aquatiques comprenant l'animal aquatique ;
le module de calcul d'angle d'incidence d'ultrasons (110) étant configuré pour calculer les premier et second angles d'incidence pour la pluralité d'animaux aquatiques ;
le module de calcul de distance entre parties (112) étant configuré pour calculer les première et seconde distances internes pour la pluralité d'animaux aquatiques ; et
le module d'informations de structure interne (114) étant configuré pour stocker la structure interne de la pluralité d'animaux aquatiques.

4. Dispositif (700) selon la revendication 3, comprenant en outre :
un module de calcul d'histogramme (702), configuré pour calculer un histogramme représentant une distribution d'un angle d'incidence et d'une distance interne de la pluralité d'animaux aquatiques à partir de la structure interne de la pluralité d'animaux aquatiques, le module d'informations de structure interne (114) étant en outre configuré pour stocker l'histogramme sous la forme d'une structure interne globale de la pluralité d'animaux aquatiques.

5. Dispositif (700) selon la revendication 4, comprenant en outre :
un module de calcul de poids moyen (704), configuré pour calculer un poids moyen de la pluralité d'animaux aquatiques à partir de l'histogramme, en extrayant de l'histogramme une pluralité de distances internes à une pluralité correspondante d'angles d'incidence, et en calculant le poids moyen sur la base de la pluralité extraite de distances internes.

6. Dispositif (700) selon la revendication 5, le module de calcul de poids moyen (704) calculant le poids moyen sur la base d'une multiplication de la pluralité de distances internes, chacune élevée à la puissance d'une valeur prédéterminée.

7. Dispositif (100, 700) selon l'une quelconque des revendications 1 à 6, comprenant en outre :
un moteur (1200) couplé au transducteur (102) et configuré pour déplacer le transducteur (102) afin de changer une direction dans laquelle le transducteur (102) émet les première et seconde ondes ultrasonores, le transducteur (102) émettant la première onde ultrasonore dans une première direction et la seconde onde ultrasonore dans une seconde direction différente de la première direction.

8. Dispositif (100, 700) selon la revendication 1, le transducteur (102) comprenant des premier et second éléments transducteurs (1302 et 1304) espacés d'une distance prédéterminée, et le premier élément transducteur (1302) émettant la première onde ultrasonore et le second élément transducteur (1304) émettant la seconde onde ultrasonore.

9. Dispositif (100, 700) selon la revendication 8, les premier et second éléments transducteurs (1302 et 1304) émettant les première et seconde ondes ultrasonores dans des directions qui sont respectivement différentes l'une de l'autre.

10. Procédé de calcul d'une structure interne d'un animal aquatique, le procédé comprenant les étapes consistant à :

émettre des première et seconde ondes ultrasonores vers un animal aquatique ;
générer des premier et second signaux d'écho à partir, respectivement, d'une réflexion des première et seconde ondes ultrasonores sur l'animal aquatique ;
calculer des premier et second angles d'incidence des première et seconde ondes ultrasonores sur l'animal aquatique, sur la base, respectivement, des premier et second signaux d'écho ;
calculer une première distance interne entre des première et seconde parties de corps de l'animal aquatique, sur la base du premier signal d'écho ;
calculer une seconde distance interne entre des première et seconde parties de corps de l'animal aquatique, sur la base du second signal d'écho ;
stocker le premier angle d'incidence et la première distance interne sous la forme d'une première paire ;
stocker le second angle d'incidence et la seconde distance interne sous la forme d'une seconde paire ; et
stocker la structure interne de l'animal aquatique, la structure interne étant basée sur les première et seconde paires ;

la seconde onde ultrasonore étant émise après la première onde ultrasonore ;

et le procédé comprenant en outre les étapes consistant à calculer le second angle d'incidence sur la base d'une première distance entre l'animal aquatique et un transducteur (102) dans le premier signal d'écho et d'une seconde distance entre l'animal aquatique et le transducteur (102) dans le second signal d'écho ; et

calculer le second angle d'incidence sur la base d'une hypothèse que les première et seconde distances varient dans le temps sur la base d'une courbe hyperbolique ;

et le premier angle d'incidence étant calculé sur la base d'une autre distance entre l'animal aquatique et le transducteur (102) dans un autre signal d'écho et de la première distance entre l'animal aquatique et le transducteur (102) dans le premier signal d'écho, l'autre signal d'écho étant généré à partir d'une réflexion sur l'animal aquatique d'une autre onde ultrasonore, émise avant la première onde ultrasonore ; et

le premier angle d'incidence étant calculé sur la base d'une hypothèse que l'autre distance et la première distance varient dans le temps sur la base d'une courbe hyperbolique.

11. Support non transitoire lisible par ordinateur sur lequel sont stockées des instructions exécutables par ordinateur qui, lorsqu'elles sont exécutées par le dispositif selon la revendication 1, amènent le dispositif à exécuter le procédé selon la revendication 10.

FIG. 1

**FIG. 2**

**FIG. 3**

401

102

θ

402

400

Tracked Fish @ $T_{n-1}$

Tracked Fish @ $T_n$

**FIG. 4A**

**FIG. 4B**

Tracked Fish @ $T_{n-1}$

Tracked Fish @ $T_n$

**FIG. 4C**

**FIG. 5**

**FIG. 6**

**FIG. 7**

800

FIG. 8

**FIG. 9A**

**FIG. 9B**

**FIG. 10A**

**FIG. 10B**

**FIG. 11**

**FIG. 12**

1302

1304

1308

1306

1310

1300

Tracked Fish @ n-1

Tracked Fish @ n

**FIG. 13**

1400

```
                        ┌─────────┐
                        │  START  │
                        └─────────┘
                             │
                             ▼
   ┌──────────────────────────────────────────────────┐
   │ Transmit first and second ultrasonic waves toward │  ⌐ 1402
   │                 an aquatic animal                  │
   └──────────────────────────────────────────────────┘
                             │
                             ▼
   ┌──────────────────────────────────────────────────┐
   │ Generate first and second echo signals from a     │  ⌐ 1404
   │ reflection of the first and second ultrasonic     │
   │ waves on the aquatic animal, respectively         │
   └──────────────────────────────────────────────────┘
                             │
                             ▼
   ┌──────────────────────────────────────────────────┐
   │ Calculate first and second incidence angles of    │  ⌐ 1406
   │ the first and second ultrasonic waves on the      │
   │ aquatic animal based on the first and second      │
   │ echo signals, respectively                        │
   └──────────────────────────────────────────────────┘
                             │
                             ▼
   ┌──────────────────────────────────────────────────┐
   │ Calculate a first internal distance between first │  ⌐ 1408
   │ and second body parts of the aquatic animal based │
   │ on the first echo signal                          │
   └──────────────────────────────────────────────────┘
                             │
                             ▼
   ┌──────────────────────────────────────────────────┐
   │ Calculate a second internal distance between      │  ⌐ 1410
   │ first and second body parts of the aquatic animal │
   │ based on the second echo signal                   │
   └──────────────────────────────────────────────────┘
                             │
                             ▼
   ┌──────────────────────────────────────────────────┐
   │ Store the first incidence angle and the first     │  ⌐ 1412
   │ internal distance as a first pair                 │
   └──────────────────────────────────────────────────┘
                             │
                             ▼
   ┌──────────────────────────────────────────────────┐
   │ Store the second incidence angle and the second   │  ⌐ 1414
   │ internal distance as a second pair                │
   └──────────────────────────────────────────────────┘
                             │
                             ▼
   ┌──────────────────────────────────────────────────┐
   │ Store an internal structure of the aquatic animal │  ⌐ 1416
   │ based on the first and second pairs               │
   └──────────────────────────────────────────────────┘
                             │
                             ▼
                        ┌─────────┐
                        │  STOP   │
                        └─────────┘
```

**FIG. 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3754374 A1

- WO 2017163904 A1

**Non-patent literature cited in the description**

- Classification of fish schools based on acoustic features associated with tilt angle. *Underwater Technology Symposium*, 05 March 2013

- Target strength spectra of tracked individual fish in schools. Fisheries Science. Japanese society of fisheries, 29 May 2015, vol. 81